# EUROPEAN PATENT APPLICATION

(11) **EP 3 480 595 A1**
(43) Date of publication of application: **08.05.2019**
(21) Application number: 17199766.1
(22) Date of filing: 02.11.2017
(51) Int. Cl.: G01N 33/543, G01N 27/327, C09D 11/52, C12Q 1/00

(54) **BIOSENSOR MANUFACTURE**

(71) Applicant: Elisha Systems Ltd., Wakefield, Yorkshire WF3 4AA (GB)
(72) Inventor: Gibson, Timothy David, Wakefield, Yorkshire WF3 4AA (GB); Sharp, Duncan William Mackenzie, Althrope, North Lincolnshire DN17 3HZ (GB)
(74) Representative: Browne, Robin Forsythe

(57) **Abstract**

The manufacture of an electrochemical sensor precursor comprising the steps of: preparing an electrically conductive composite comprising an aqueous mixture of:
polycationic conductive polymer particles;
an anionic polyelectrolyte;
and
an optional buffer;
applying the mixture to a region of an electroconductive surface; and
removing water to allow adhesion of the composite to the region of the surface.

## Description

This invention relates to biosensors and methods and materials for use in manufacture of biosensors, particularly but not exclusively for automated or robotic manufacture of electrochemical sensors which incorporate biological materials.

According to a first aspect of the present invention a method of manufacture of an electrochemical sensor precursor comprises the steps of:
preparing an electrically conductive composite comprising an aqueous mixture of:
   polycationic conductive polymer particles;
   an anionic polyelectrolyte; and
   an optional buffer;
   applying the mixture to a region of an electroconductive surface; and
   removing water to allow adhesion of the composite to the region of the surface.

The composite may further comprise a biological affinity agent; and

The polymer may comprise particles having a dimension of about 5 to 750nm, optionally about 10 to about 500nm, optionally about 30 to about 400nm, optionally about 70 to about 400nm. The particles may comprise nanoparticles, nanotubes or mixtures thereof.

The conductive polymer may be selected from the group consisting of: poly(3,4-ethylenedioxythiophene) (PEDOT) and related polymers e.g. poly (3,4-propylenedioxythiophene (PProDOT), and mixtures thereof. Alternative conductive polymers include polypyrrole and substituents thereof for example, poly (pyrrole-3-carboxilic acid), poly(1-cyanoethyl)pyrrole and poly(n-methyl)pyrrole), poly(polycarbazole and substituents thereof,
Alternative conductive polymers may be selected from the group consisting of polyaniline (PANI), and substituted polyanilines for example poly(2-aminobenzylamine), poly(2-aminobenzoic acid) and poly(2-methyl-aniline).

The anionic polyelectrolyte may be selected from the group consisting of: polystyrene sulphonic acid (PSS), alginic acid, poly(acrylic acid), poly(vinyl sulphonic acid), dextran sulphate, heparin, and multi-anionic dyestuffs including direct red 80, direct blue 15, direct blue 14, direct blue 1, direct blue 6, acid blue 29, acid black 1, acid red 27, acid red 113 and mixtures thereof.

Exemplary polyelectrolytes have 20 or more acid groups and may have a molecular weight greater than 20,000, for example 40,000 or greater.

Advantageous conductive polymers are selected from sulphur-containing polyheterocyclic compounds. Sulphur-containing cyclic coating compounds have been reported to form strong bonds to gold, platinum or other noble metal electrodes, with PEDOT being one of the strongest.

Composites of PEDOT and PSS are commercially available, for example from Bayer AG under the trade mark Baytron. A combination of PSS which is water soluble, with the insoluble PEDOT may give a material which acts as a solution but which comprises a dispersion of nanoparticles having various Redox states dependent on pH and other conditions. The measurement of particle size in 50mM MOPS buffer at pH 7.05 provided particles of two sizes at about 38nm and about 825nm, although the larger particles could be aggregates. Dimensions of about 70nm to about 400nm have been obtained. Commercial PEDOT/PSS composites for example as available from Sigma Aldrich, comprise 3 to 4 wt% in water at pH 5. Adding 50mM, pH 7.5 MOPS buffer may effectively adjust the pH of the aqueous composite to 7. This may be a key step to incorporate active biological molecules.

Composites of PEDOT and PSS have an advantage of being very stable in electrochemical applications and may be used for more than 100 cycles without degradation.

The composites may be transparent, facilitating use in photochromic devices.

In use a biological affinity agent is applied to the region of the precursor surface to form an electrochemical sensor.

The addition of a biological affinity agent, for example an antibody protein to a PSS solution forms a complex due to electrostatic interaction between the protein and the PSS molecules to give a protein-polyelectrolyte complex. Addition of an antibody protein to a PEDOT:PSS nanoparticle suspension appears to form a complex mixture of the protein and the anionic (PSS) and cationic polymer (PEDOT) nanoparticles. It has been found that addition of the protein/PEDOT/PSS nanoparticle complex to an electroconductive surface, for example a screen printed gold electrode, has the unexpected result that the complex adheres strongly to the surface and is not removed by washing with distilled water, buffer solutions or buffered non-ionic detergents such Tween 20 at a concentration of 0.1v/v%. Furthermore addition of multiple layers of the protein/PEDOT/PSS nanoparticle complex to a surface is possible to allow building up of a strongly adherent coating.

The biological affinity agent may be an antibody (polyclonal or monoclonal), antibody fragments such as F(ab) fragments, F(ab)₂ fragments or nanobodies (cloned Fv fragments), a binding protein such as cellulose binding protein, a nucleic acid such as DNE, RNA or composites thereof, an antibody biomimic such as an Affimer, Darpin or other conserved scaffold proteins, a nanocomposite material consisting of an aggregate or covalent complex of any of the above with a polymer molecule or nanoparticle (e.g. a gold nanoparticle-Affimer complex or gold nanoparticle-antibody complex or a dendrimer nanocomposite).

The method may further comprise the steps of application of aliquots of the aqueous composite to a selected region of the surface of an electrode, allowing the composite to dry, forming a coating on the electrode. The step of adding the aqueous composite may be repeated as necessary to build up a layer on the surface.

The composite may be applied to the substrate by drop casting, printing, spraying, using an air jet or other deposition method.

The electroconductive surface may be a region of an electroconductive electrode.

According to a second aspect of the present invention an electrochemical sensor precursor comprises an electroconductive surface comprising a plurality of electrodes, one electrode including a layer of an electrically conductive composite comprising polycationic conductive polymer particles and an anionic polyelectrolyte.

The electroconductive surface which may comprise an electrode may be composed of screen printed or ink jet printed carbon or noble metal, for example, gold or platinum. Graphene ink coated electrodes may also be used.

Photolithographically or sputtered gold or platinum electrodes may be employed. However the use of photolithographically or sputtered gold electrodes may not be preferred on account of their high cost.

Use of a biosensor in accordance with the present invention confers numerous advantages. The biosensor has been found to function well in both Faradaic and non-Faradaic buffers, for example ferri/ferrocyanide buffer or in non-conductive buffers such as phosphate buffered saline (PBS). This unexpectedly provides the advantage that it allows an analyte solution to be added directly without modifying using a buffer or other additive solution. Also electropolymerization of the polymer coating onto the conductive surface is avoided, facilitating large scale robotic manufacture of arrays of the biosensors.

The biosensors may be employed in apparatus using AC impedance interrogation or other methods including cyclic voltammetry, differential pulse voltammetry, chronopotentiometry and open circuit potentiometry.

The invention is further described by means of examples but not in any limitative sense with reference to the accompanying drawings of which:
Figure 1 shows a calibration curve for a PEDOT:PSS Troponin-I conjugate biosensor;
Figure 2 shows the degree of luminescence of a PEDOT:PSS conjugate biosensor;
Figure 3 shows a calibration curve for a PEDOT:PSS Bacteria conjugate biosensor;
Figure 4 shows a calibration curve for a PEDOT:PSS PBP2a conjugate biosensor.

### Example 1 - Preparation of PEDOT: PSS conjugate and consequent biosensor for Troponin-I measurement.

A conjugate was prepared by the following method:
A 1 in 10 dilution of PEDOT:PSS nanoparticles (Sigma Aldrich catalogue number 655201, high conductivity grade 3-4%w/v dispersion in water) into 50mM MOPS buffer pH 7.05 was first made.

Then 10µl of polyclonal anti-troponin-I (1mg per ml in 50mM MOPS buffer pH 7.05) was added to 40µl of the 1/10 diluted PEDOT:PSS suspension, to give an antibody/PEDOT:PSS complex containing 200µg per ml antibody - specific antibody complex or SAC.

A second antibody complex was prepared using polyclonal anti-digoxin (2mg per ml in 100mM PBS buffer pH 7.1) adding to 90µl of the 1/10 diluted PEDOT:PSS suspension, to give an antibody/PEDOT:PSS complex containing 200µg per ml antibody - non-specific antibody complex or NSAC.

0.4µl of SAC was deposited on the upper surface of working electrode 2 (WE2) of a dual gold electrode (DropSens X222OAT) and 0.4µl or NSAC was deposited on the upper surface of working electrode 1 (WE1) of a dual gold electrode, then the deposited suspensions were dried on a hot plate set at 37°C. This procedure was repeated 2 further times to give a thin layer of antibody/PEDOT:PSS complex adhering strongly to the surface of the gold electrodes.

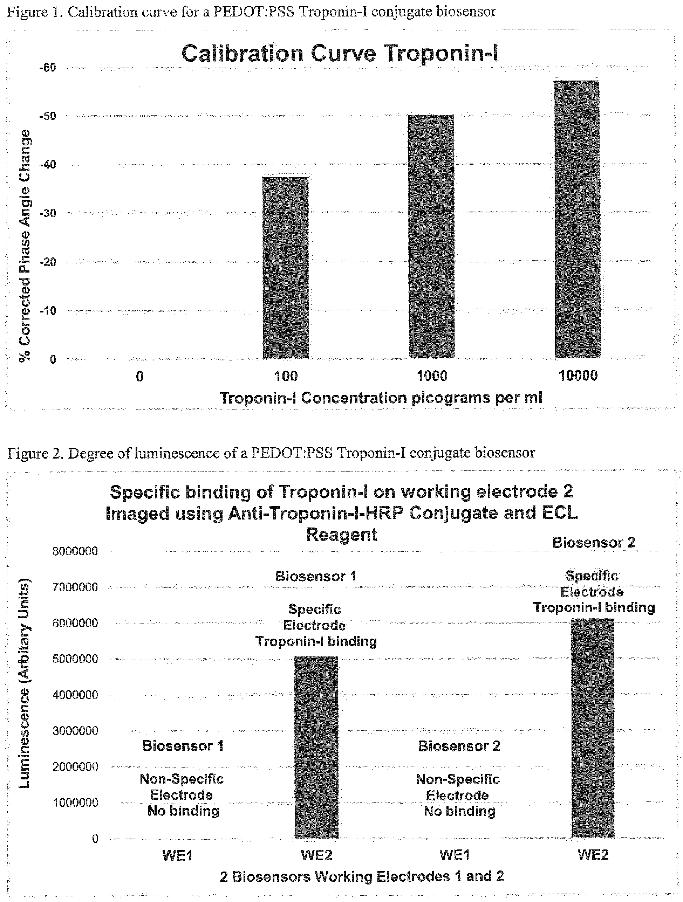

Incubation of solutions of Troponin-I onto both electrode surfaces for 5 minutes resulted in measurable changes to the AC impedance when they were scanned between 1000Hz down to 1Hz in PBS (non-Faradaic buffer), indicating affinity binding took place. Any non-specific binding that occurred was corrected for by the NSAC on WE1 by subtracting the non-specific signal obtained from the signal from WE2. The differential signal (WE2-WE1) was a measure of the concentration of Troponin-I. A concentration dependant calibration curve is depicted in Figure 1.

### Example 2 - Demonstration of adherence of protein to the electrode surface

Strong adherence of protein:PEDOT:PSS nanoparticle complexes to electrode surfaces and specific binding capabilities was demonstrated using an optical imaging technique. The electrode surfaces were prepared using a polyclonal Anti-Troponin-I added to PEDOT:PSS nanoparticles in MOPS buffer (45mM, pH 7.05). Three aliquots of 0.2µl were drop cast onto the electrode surfaces and dried at 37°C for 2-3 minutes between aliquots. The control electrode was prepared using polyclonal Anti-Digoxin in exactly the same manner. The biosensors thus prepared were incubated with analyte (Troponin-I) and then washed with buffered 0.1% v/v Tween 20 to remove any non-specific bound analyte. Then an Anti-Troponin-HRP conjugate was used to label the bound analyte, washed again with buffered 0.1 % v/v Tween 20 to remove any non-specific bound conjugate. Pierce ECL reagent was then added and the electrodes imaged in a GeneSys imager. The surfaces luminesced due to the bound HRP label. A higher degree of luminescence indicated bound analyte, Figure 2.

### Example 3 - Preparation of PEDOT: PSS conjugate and consequent biosensor for Streptococcus pyogenes measurement.

A conjugate was prepared by the following method:
A 1 in 10 dilution of PEDOT:PSS nanoparticles (Sigma Aldrich catalogue number 655201, high conductivity grade 3-4%w/v dispersion in water) into 50mM MOPS buffer pH 7.05 was first made.

Then 10µl of polyclonal anti- *Streptococcus pyogenes* (1mg per ml in 50mM MOPS buffer pH 7.05) was added to 40µl of the 1/10 diluted PEDOT:PSS suspension, to give an antibody/PEDOT:PSS complex containing 200µg per ml antibody-specific antibody complex or SAC.

A second antibody complex was prepared using polyclonal anti-digoxin (2mg per ml in 100mM PBS buffer pH 7.1) adding to 90µl of the 1/10 diluted PEDOT:PSS suspension, to give an antibody/PEDOT:PSS complex containing 200µg per ml antibody - non-specific antibody complex or NSAC.

0.2µl of 1 in 10 dilution of PEDOT:PSS nanoparticles was deposited on the upper surface of both working electrodes (WE1 and WE2) of a dual gold electrode (DropSens X222OAT) and dried on a hot plate set at 37°C. When dry 1µl of NSAC was deposited on the upper surface of working electrode 1 (WE1) and 1µl of SAC was deposited on the upper surface of working electrode 2 (WE2) of a dual gold electrode, then the deposited suspensions were dried on a hot plate set at 37°C. This 2 stage procedure gave a thin layer of antibody/PEDOT:PSS complex adhering strongly to the surface of the gold electrodes.

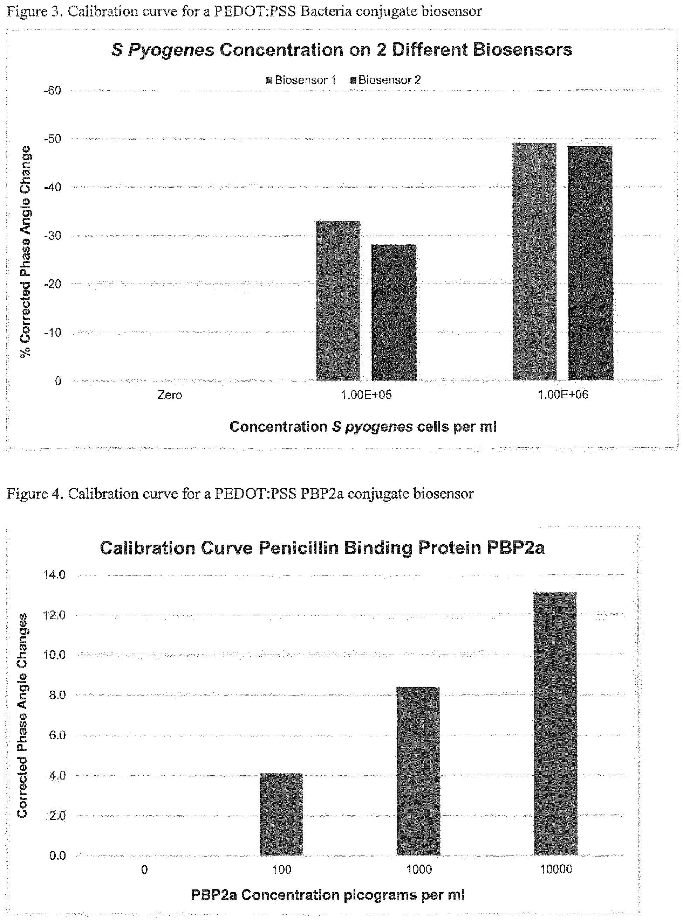

Incubation of suspensions of *Streptococcus pyogenes* onto both electrode surfaces for 15 minutes resulted in measurable changes to the AC impedance when they were scanned between 1000Hz down to 1 Hz in ferri/ferrocyanide buffer (Faradaic buffer), indicating affinity binding took place. Any non-specific binding that occurred was corrected for by the NSAC on WE1 by subtracting the non-specific signal obtained from the signal from WE2. The differential signal (WE2-WE1) was a measure of the concentration of *Streptococcus pyogenes.* A concentration dependant calibration curve is depicted in Figure 3.

### Example 4 - Preparation of PEDOT: PSS conjugate and consequent biosensor for Penicillin Binding Protein 2a (PBP2a) measurement. PBP2a is a biomarker for MRSA.

A conjugate was prepared by the following method:
A 1 in 10 dilution of PEDOT:PSS nanoparticles (Sigma Aldrich catalogue number 655201, high conductivity grade 3-4%w/v dispersion in water) into 50mM MOPS buffer pH 7.05 was first made.

Then 10µl of polyclonal anti-PBP2aI (1mg per ml in 50mM MOPS buffer pH 7.05) was added to 40µl of the 1/10 diluted PEDOT:PSS suspension, to give an antibody/PEDOT:PSS complex containing 200µg per ml antibody-specific antibody complex or SAC.

A second antibody complex was prepared using polyclonal anti-digoxin (2mg per ml in 100mM PBS buffer pH 7.1) adding to 90µl of the 1/10 diluted PEDOT:PSS suspension, to give an antibody/PEDOT:PSS complex containing 200µg per ml antibody-non-specific antibody complex or NSAC.

0.4µl of SAC was deposited on the upper surface of working electrode 2 (WE2) of a dual gold electrode (DropSens X222OAT) and 0.4µl or NSAC was deposited on the upper surface of working electrode 1 (WE1) of a dual gold electrode, then the deposited suspensions were dried on a hot plate set at 37°C. This procedure was repeated 2 further times to give a thin layer of antibody/PEDOT:PSS complex adhering strongly to the surface of the gold electrodes.

Incubation of solutions of PBP2a onto both electrode surfaces for 5 minutes resulted in measurable changes to the AC impedance when they were scanned between 1000Hz down to 1Hz in ferri/ferrocyanide buffer (Faradaic buffer), indicating affinity binding took place. Any non-specific binding that occurred was corrected for by the NSAC on WE1 by subtracting the non-specific signal obtained from the signal from WE2. The differential signal (WE2-WE1) was a measure of the concentration of PBP2a. A concentration dependant calibration curve is depicted in Figure 4.

## Claims

1. A method of manufacture of an electrochemical sensor precursor comprising the steps of:
preparing an electrically conductive composite comprising an aqueous mixture of:
polycationic conductive polymer particles;
an anionic polyelectrolyte; and
an optional buffer;
applying the mixture to a region of an electroconductive surface; and
removing water to allow adhesion of the composite to the region of the surface.

2. A method as claimed in claim 1, wherein the composite further comprises a biological affinity agent.

3. A method as claimed in claim 1 or 2, wherein the polymer comprises particles having a dimension of 5 to 750nm.

4. A method as claimed in claim 3, wherein the particles have a dimension of 10 to 500nm.

5. A method as claimed in claim 4, wherein the particles have a dimension of 30 to 400nm.

6. A method as claimed in claim 5, wherein the particles have a dimension of 70 to 400nm.

7. A method as claimed in any preceding claim, wherein the particles comprise nanoparticles, nanotubes or mixtures thereof.

8. A method as claimed in any preceding claim, wherein the conductive polymer is related from the group consisting of: poly (3,4-ethylenedioxythiophene) (PEDOT) and related polymers e.g. poly (3,4-propylenedioxythiophene (PProDOT), polypyrrole and substituted polypyrroles, poly(pyrrole-3-carboxylic acid), poly(1-cyanoethyl)pyrrole and poly(n-methyl)pyrrole), and polycarbazole and substituted polycarbazoles.

9. A method as claimed in any of claims 1 to 7, wherein the conductive polymer is selected from the group consisting of: polyaniline (PANI) and mixtures thereof; substituted polyanilines for example, poly(2-aminobenzylamine), poly(2-aminobenzoic acid) and poly(2-methyl-aniline).

10. A method as claimed in any preceding claim, wherein the anionic polyelectrolyte is selected from the group consisting of:
polystyrene sulphonic acid (PSS), alginic acid and mixtures thereof;
and multi-anionic dyestuffs including: direct red 80, direct blue 15, direct blue 14, direct blue 1, direct blue 6, acid blue 29, acid black 1, acid red 27, acid red 113 and mixtures thereof.

11. A method as claimed in any of claims 2 to 10, wherein the biological affinity agent is selected from the group consisting of:
A polyclonal antibody or monoclonal antibody, antibody fragments, for example: F(ab) fragments, F(ab)₂ fragments or nanobodies (cloned Fv fragments), a binding protein, for example: cellulose binding protein, a nucleic acid such as DNE, RNA or composites thereof, an antibody biomimic, for example: an Affimer, Darpin or other conserved scaffold proteins

12. A method as claimed in any preceding claim, comprising the steps of application of aliquots of the aqueous composite to a selected region of the surface of an electrode, allowing the composite to dry, forming a coating on the electrode.

13. A method as claimed in claim 12, wherein the composite is applied by drop casting, robotically controlled liquid deposition, printing or spraying.

14. An electrochemical sensor precursor comprising an electroconductive surface comprising a plurality of electrodes, one electrode including a layer of electrically conductive composite comprising polycationic conductive polymer particles and an anionic polyelectrolyte.

15. An electrochemical sensor precursor as claimed in claim 14, wherein the electroconductive surface comprises an electrode composed of screen printed or ink jet printed carbon or graphene ink.
